# EUROPEAN PATENT APPLICATION

(11) **EP 2 243 429 A1**
(43) Date of publication of application: **27.10.2010**
(21) Application number: 10158101.5
(22) Date of filing: 29.03.2010
(51) Int. Cl.: A61B 8/00

(54) **Ultrasonic diagnostic apparatus**

(30) Priority: 22.04.2009 KR 20090035122
(71) Applicant: MEDISON CO., LTD., Gangwon-do 250-875 (KR)
(72) Inventor: Shim, Jae Yoon, Seoul (KR)
(74) Representative: Schmid, Wolfgang

(57) **Abstract**

An ultrasonic diagnostic apparatus (100; 200) is disclosed. The ultrasonic diagnostic apparatus (100; 200) includes a main body (110), a receiving part (120) provided to the main body (110), and an arch-shaped support (130; 230) mounted on the main body (110) to form the receiving part (120) on the main body (110). The main body (110) is provided at an upper portion thereof with the arch-shaped support (130; 230) so that the receiving part (120) is defined between the main body (110) and a display unit (140) and can receive peripheral devices, such as an echo-printer, VCR, and the like, which are connected to the ultrasonic diagnostic apparatus, and other articles, thereby providing convenience in maintenance and storage of the peripheral devices and the like.

## Description

### BACKGROUND OF THE INVENTION

### 1. Field of the Invention

The present invention relates to an ultrasonic diagnostic apparatus and, more particularly, to an ultrasonic diagnostic apparatus that generates internal images of a target using ultrasound waves.

### 2. Description of the Related Art

An ultrasonic diagnostic apparatus is an inspection apparatus that generally irradiates an ultrasound signal to a target and obtains an internal image of the target by converting a time difference between sending the ultrasound signal and receiving an echo-signal into a distance. Since the ultrasonic diagnostic apparatus employs ultrasound waves that are harmless to the human body, it is particularly useful for medical inspection and has been widely used for detection of foreign matter in a body, determination as to a degree of damage, observation of cancers or fetuses, and the like.

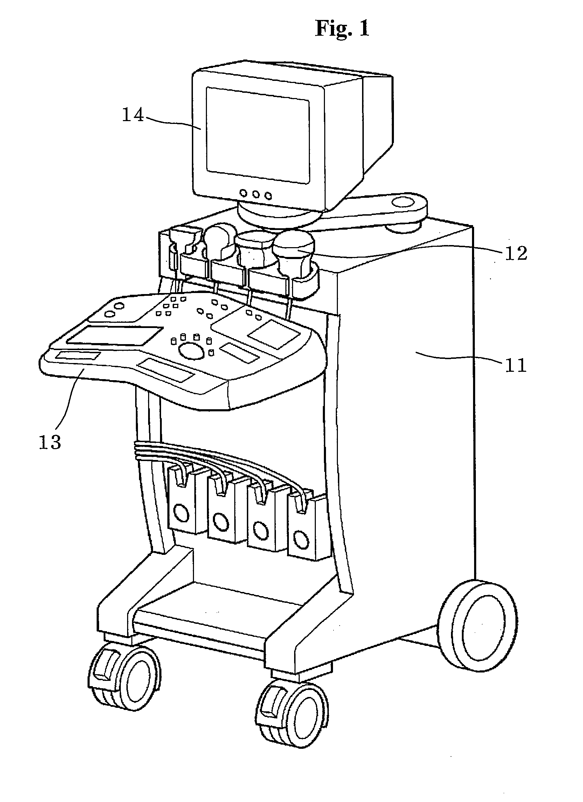

Fig. 1 is a perspective view of a typical ultrasonic diagnostic apparatus.

Referring to Fig. 1, the ultrasonic diagnostic apparatus includes a cart-shaped body 11 that receives main components of the apparatus, a probe 12 that transmits and receives ultrasound waves, a control panel 13 that includes various switches and keys to input commands for manipulation of the apparatus, and a display unit 14 that displays images of ultrasonic diagnosis results.

When using the apparatus for ultrasound diagnosis of a target, an operator moves the probe 12 with one hand while keeping the probe 12 in contact with a surface of the target, and manipulates the control panel 13 with the other hand. Then, the result of the ultrasound diagnosis is provided as an image through the display unit 14.

In such an ultrasound diagnostic apparatus, however, since there is no space for receiving peripheral devices connected to the diagnostic apparatus, a separate holder is inconveniently needed to receive the peripheral devices. Therefore, there is a need to solve such a problem.

### SUMMARY OF THE INVENTION

The present invention is conceived to solve the problem of the related art, and one aspect of the invention is to provide an improved ultrasonic diagnostic apparatus configured to eliminate inconvenience caused by receiving peripheral devices in a separate holder or the like.

In accordance with one aspect of the invention, an ultrasonic diagnostic apparatus includes: a main body; a receiving part provided to the main body; and an arch-shaped support mounted on the main body to form the receiving part on the main body.

The arch-shaped support may include a first support mounted on the main body and extending in a first direction; a second support extending in a second direction from the first support; and a third support extending in a third direction from the second support to be mounted on the main body.

The arch-shaped support may be detachably mounted to the main body.

The ultrasonic diagnostic apparatus may further include a display unit support provided to the arch-shaped support to support a display unit.

The display unit support may further include a mounting portion provided on the arch-shaped support and a support member mounted on the mounting portion to support the display unit.

The ultrasonic diagnostic apparatus may further include a tray provided to the arch-shaped support.

The arch-shaped support may further include a tray support supporting the tray.

The tray support may be detachably attached to the arch-shaped support.

The receiving part may further include a detachment preventing portion preventing detachment of an article from the receiving part.

### BRIEF DESCRIPTION OF THE DRAWINGS

The above and other aspects, features and advantages of the invention will become apparent from the following description of embodiments given in conjunction with the accompanying drawings, in which:
- Fig. 1: is a perspective view of a typical ultrasonic diagnostic apparatus;
- Fig. 2: is a perspective view of an ultrasonic diagnostic apparatus in accordance with one embodiment of the present invention;
- Fig. 3: is an exploded perspective view of the ultrasonic diagnostic apparatus shown in Fig. 2;
- Fig. 4: is a perspective view of an ultrasonic diagnostic apparatus in accordance with another embodiment of the present invention; and
- Fig. 5: is an exploded perspective view of the ultrasonic diagnostic apparatus shown in Fig. 4.

### DETAILED DESCRIPTION OF THE EMBODIMENT

Embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the invention into account and can be changed according to the custom or intention of users or operators. Therefore, definition of the terms should be made according to overall disclosures set forth herein.

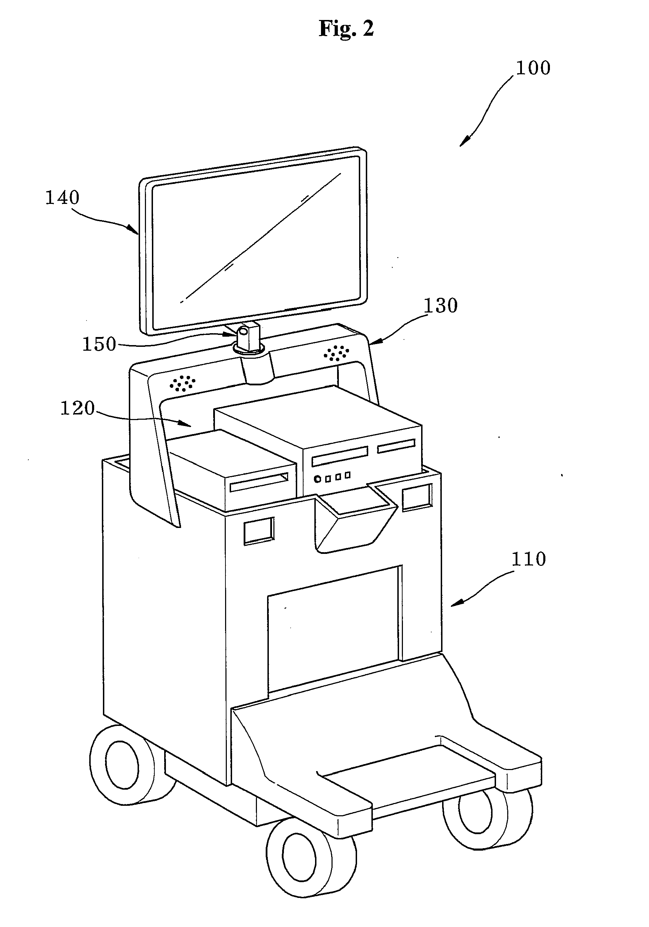

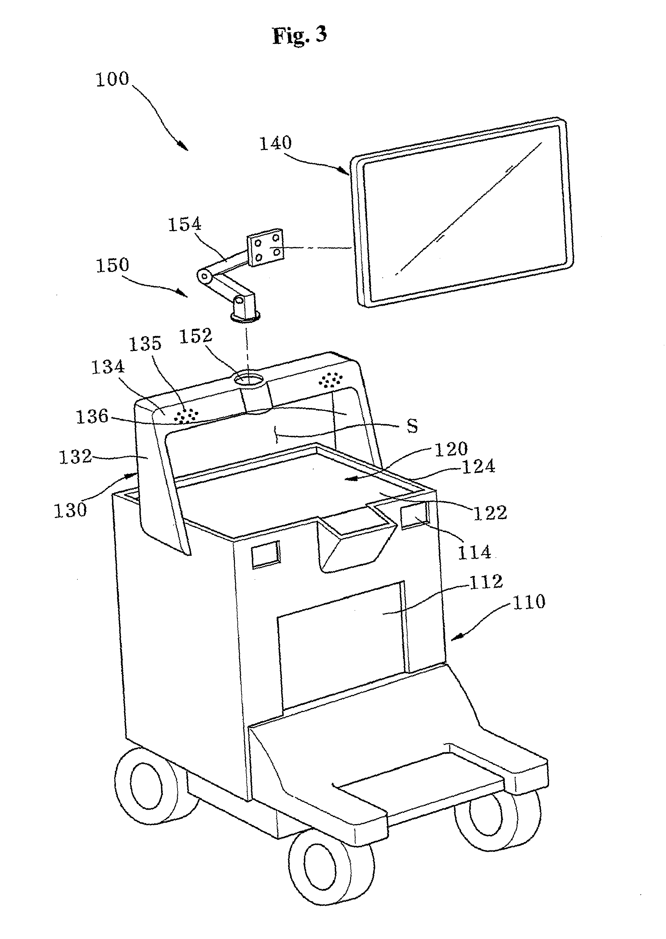

Fig. 2 is a perspective view of an ultrasonic diagnostic apparatus in accordance with one embodiment of the invention, and Fig. 3 is an exploded perspective view of the ultrasonic diagnostic apparatus shown in Fig. 2.

Referring to Figs. 2 and 3, the ultrasonic diagnostic apparatus 100 according to this embodiment includes a main body 110, a receiving part 120, and an arch-shaped support 130.

The main body 110 includes a beam former (not shown) for transmit-focusing ultrasonic signals transmitted through a probe (not shown) and receive-focusing ultrasonic signals received through the probe, a data creator (not shown) for creating frame data based on the signals output from the beam former, a processor (not shown) for generating a two-dimensional or three-dimensional interior image of a target based on the frame data, a storage (not shown) for storing data, and a control panel (not shown) for operating the ultrasonic diagnostic apparatus 100 or selecting functions.

Furthermore, the main body 110 may be provided with a probe connector panel 112 to which a connector of the probe is connected so that the probe is connected to the main body 110 therethrough, and an electrocardiogram (ECG) connector panel 114 to which a connector of an ECG electrode for ECG measurement is connected so that the ECG electrode is connected to the main body 110 therethrough.

The main body 110 is formed with the receiving part 120. Specifically, the main body 110 is formed at an upper portion thereof with the receiving part 120 that includes a bottom surface 122 formed on the upper surface of the main body 110 and an receipt space S defined above the bottom surface 122.

The receiving part 120 can receive a variety of articles. For example, the receiving part 120 can receive peripheral devices, such as an echo-printer, VCR, and the like, which are connected to the ultrasonic diagnostic apparatus 100.

In this embodiment, the receiving part 120 may further include a detachment prevention portion 124. The detachment prevention portion 124 is formed along a periphery of the receiving part 120 and extends upward from the bottom surface 122 along an edge of the bottom surface 122, thereby preventing deviation of the article from the receiving part 120.

The arch-shaped support 130 is mounted on the main body 110. Specifically, the arch-shaped support 130 is mounted on the upper portion of the main body 110 to define the receiving part 120. The arch-shaped support 130 includes a first support 132, a second support 134, and a third support 136.

The first support 132 is coupled to the main body 110 and extends in a first direction. In this embodiment, the first support 132 is illustrated as being coupled at one end thereof to the upper portion of one side of the main body 110 and extending upward in the vertical direction with respect to the bottom surface 122 of the receiving part 120.

The second support 134 extends in a second direction from the first support 132 towards the other side of the main body 120 to be parallel to, for example, the bottom surface 122 of the receiving part 120. The third support 136 extends in a third direction from the second support 134, for example, downward in the vertical direction with respect to the second support 134. The third support 136 extending in the third direction is coupled at one end thereof to the upper portion of the other side of the main body 110.

The first support 132 and the third support 136 may be detachably coupled at the ends thereof to the upper portion of the main body 110. In other words, the arch-shaped support 130 may be detachably coupled to the main body 110.

As such, the arch-shaped support 130 including the first to third supports 132, 134, 136 substantially has a "n " shape. The upper surface of the main body 110 is separated a predetermined distance from the second support 134 by the arch-shaped support 130, thereby allowing the receiving part 120 composed of the bottom surface 122 and the receipt space S to be defined between the upper surface of the main body 110 and the arch-shaped support 130.

In this embodiment, the ultrasonic diagnostic apparatus 100 may further include a sound output unit 135. The sound output unit 135 outputs sound generated in the main body 110 to the outside. In this embodiment, the sound output unit 135 is illustrated as being integrally formed with the arch-shaped support 130.

The sound output unit 135 may include a speaker (not shown) for outputting a sound, which is disposed inside the arch-shaped support 130, for example, inside the second support 134 and connected to the main body 110 through the interior of the arch-shaped support 130. The second support 134 may be formed with one or plural through-holes (not shown) through which the speaker communicates with the outside of the arch-shaped support 130.

In this embodiment, the ultrasonic diagnostic apparatus 100 may further include a display unit 140. The display unit 140 serves to display ultrasound images generated by the main body 110, operation states of the ultrasonic diagnostic apparatus 100, preset information for operating the ultrasonic diagnostic apparatus 100, and the like.

The display unit 140 may be directly mounted to the arch-shaped support 130 or may be mounted thereto via a display unit support 150. In this embodiment, the display unit 140 will be illustrated as being mounted to the arch-shaped support 130 via the display unit support 150.

The display unit support 150 is provided to the arch-shaped support 130 and supports the display unit 140. The display unit support 150 includes a mounting portion 152 and a support member 154.

The mounting portion 152 is provided on the arch-shaped support 130. In this embodiment, the mounting portion 152 is formed on the second support 134 of the arch-shaped support 130. The mounting portion 152 may have a concave shape such that the support member 154 described below can be inserted into the mounting portion 152 or may have a convex shape such that the mounting portion 152 can be inserted into the support member 154.

The support member 154 is mounted on the mounting portion 152. The support member 154 is coupled at one side thereof to the mounting portion 152 and at the other side thereof to the display unit 140 to support the display unit 140. The support member 154 may be secured to the mounting portion 152 or may be detachably coupled thereto. The display unit 140 is coupled to the arch-shaped support 130 via the support member 154.

In the ultrasonic diagnostic apparatus 100 according to the embodiment, the arch-shaped support 130 is provided to the upper portion of the main body 110 to form the receiving part 120 between the main body 110 and the display unit 140, so that the receiving part 120 receives peripheral devices, such as an echo-printer, VCR, and the like, which are connected to the ultrasonic diagnostic apparatus 100, and other articles, thereby providing convenience in maintenance and storage of the peripheral devices and the like.

In addition, according to this embodiment, the arch-shaped support 130 and the display unit 140 may be detachably mounted to the ultrasonic diagnostic apparatus 100, so that the arch-shaped support 130 or the display unit 140 can be separated therefrom as needed, thereby reducing the overall size of the ultrasonic diagnostic apparatus while facilitating storage of components thereof.

Moreover, in the ultrasonic diagnostic apparatus 100 according to this embodiment, the sound output unit 135 is integrated to the arch-shaped support 130, thereby reducing the number of components and the overall size of the apparatus.

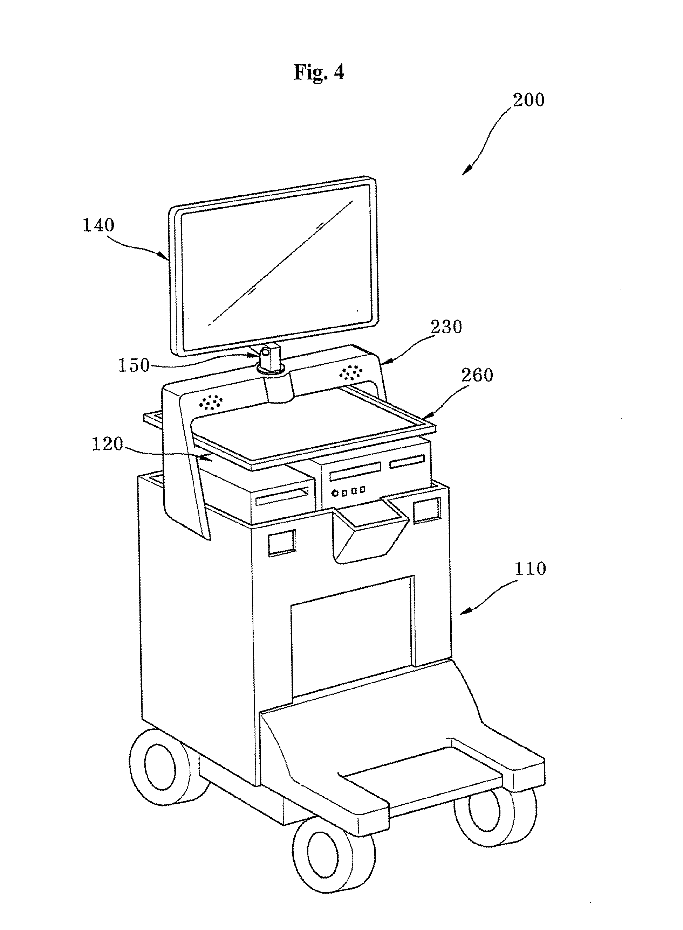

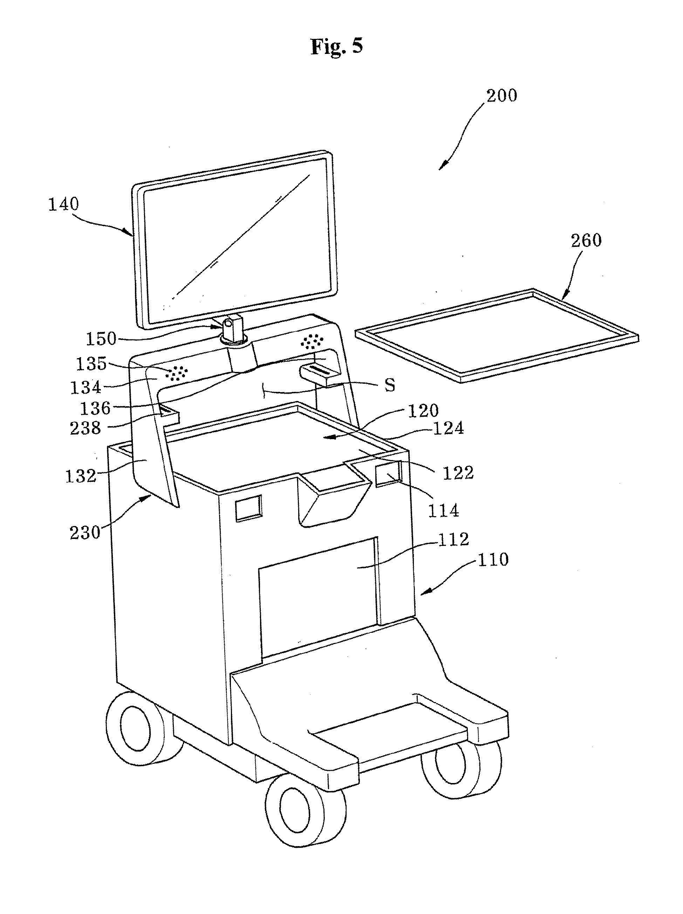

Fig. 4 is a perspective view of an ultrasonic diagnostic apparatus in accordance with another embodiment of the invention, and Fig. 5 is an exploded perspective view of the ultrasonic diagnostic apparatus shown in Fig. 4.

The same or similar components to those of the above embodiment will be denoted by the same reference numerals and a detailed description thereof will be omitted herein.

Referring to Figs. 4 and 5, the ultrasonic diagnostic apparatus 200 according to this embodiment includes a main body 110, a receiving part 120, an arch-shaped support 230, and a tray 260.

The tray 260 is provided to the arch-shaped support 230. The tray 260 may have a polygonal shape including a rectangular shape, or may have a circular shape or elliptical shape. The tray 260 is provided to the arch-shaped support 230 to be disposed above a receipt space S of the receiving part 120.

In order to mount the tray 260 to the arch-shaped support 230, the arch-shaped support 230 is formed with a tray support 238 that supports the tray 260. The tray support 238 may protrude from opposite inner sides of first and third supports 132 and 136. The tray support 238 supports a lower side of the tray 260 so that the tray 260 can be mounted to the arch-shaped support 230 while being disposed above the receipt space S.

The tray 260 may be used to receive a small article such as Tee Probe or the like, and may be secured to the tray support 238 or may be detachably coupled thereto.

When the tray 260 is detachably coupled to the tray support 238, a user can attach the tray 260 to the arch-shaped support 230 to place a small article on the tray 260 or can separate the tray 260 therefrom to place a larger article on the receiving part 120, so that the receipt space S of the receiving part 120 can be more efficiently used.

As apparent from the above, according to the embodiments, the main body of the apparatus is provided at the upper portion thereof with the arch-shaped support so that the receiving part is defmed between the main body and the display unit and can receive peripheral devices, such as an echo-printer, VCR, and the like, which are connected to the ultrasonic diagnostic apparatus, and other articles, thereby providing convenience in maintenance and storage of the peripheral devices and the like.

Further, according to the embodiment, the arch-shaped support and the display unit are detachably provided to the apparatus so that they can be separated therefrom, as needed, to reduce the overall size of the apparatus, thereby facilitating maintenance of components of the apparatus.

Further, according to the embodiment, the tray is detachably coupled to the tray support, thereby enabling more efficient use of the receipt space in the receiving part.

Although some embodiments have been provided to illustrate the invention in conjunction with the drawings, it will be apparent to those skilled in the art that the embodiments are given by way of illustration only, and that various modifications and equivalent embodiments can be made without departing from the spirit and scope of the invention. The scope of the invention should be limited only by the accompanying claims.

## Claims

1. An ultrasonic diagnostic apparatus (100; 200), **characterized by** comprising:
a main body (110);
a receiving part (120) provided to the main body (110); and
an arch-shaped support (130; 230) mounted on the main body (110) to form the receiving part (120) on the main body (110).

2. The apparatus (100; 200) according to claim 1, **characterized in that** the arch-shaped support (130; 230) comprises:
a first support (132) mounted on the main body (110) and extending in a first direction;
a second support (134) extending in a second direction from the first support (132); and
a third support (136) extending in a third direction from the second support (134) to be mounted on the main body (110).

3. The apparatus (100; 200) according to claim 1, **characterized in that** the arch-shaped support (130; 230) is detachably mounted to the main body (110).

4. The apparatus (100; 200) according to claim 1, **characterized by** further comprising:
a display unit support (150) provided to the arch-shaped support (130; 230) to support a display unit (140).

5. The apparatus (100; 200) according to claim 4, **characterized in that** the display unit support (150) comprises:
a mounting portion (152) provided on the arch-shaped support (130; 230); and
a support member (154) mounted on the mounting portion (152) to support the display unit (140).

6. The apparatus (200) according to claim 1, **characterized by** further comprising:
a tray (260) provided to the arch-shaped support (230).

7. The apparatus (200) according to claim 6, **characterized in that** the arch-shaped support (230) further comprises a tray support (238) supporting the tray (260).

8. The apparatus (200) according to claim 7, **characterized in that** the tray support (238) is detachably attached to the arch-shaped support (230).

9. The apparatus (100; 200) according to any one of claim 1 to 8, **characterized in that** the receiving part (120) further comprises a detachment preventing portion (124) preventing detachment of an article therefrom.
